# EUROPEAN PATENT SPECIFICATION

(11) **EP 3 799 842 B1**
(45) Date of publication and mention of the grant of the patent: **08.11.2023**
(21) Application number: 20199127.0
(22) Date of filing: 30.09.2020
(51) Int. Cl.: A61F 2/46, A61B 17/00, A61F 2/30

(54) **MEDICAL IMPLANT EXTRACTOR**
EXTRAKTOR FÜR MEDIZINISCHE IMPLANTATE
EXTRACTEUR D'IMPLANTS MÉDICAUX

(30) Priority: 01.10.2019 US 201962908810 P; 11.02.2020 US 202016787815
(43) Date of publication of application: 07.04.2021
(73) Proprietor: Shukla Medical, St. Petersburg, FL 33709 (US)
(72) Inventor: BRAVMAN, Jonathan Todd, Golden, CO 80403 (US); SWEITZER, Zachary Robert, Keyport, NJ 07738 (US)
(74) Representative: Swea IP Law AB

(56) References cited:
- EP-A1- 2 626 043
- EP-A1- 2 644 163
- US-A1- 2012 004 664
- US-A1- 2013 282 018
- US-A1- 2014 311 299
- US-B1- 9 402 740

## Description

The present invention is defined in claim 1 and relates to a surgical extraction device and, more specifically, to a device for extracting medical implants from bone.

### BACKGROUND OF THE DISCLOSURE

Medical implant extraction devices for extracting implants from bone assume various forms and modes of operation. Some include devices that clamp an implant prior to implant extraction. Such devices involve moving clamp parts that oftentimes require two-handed operation: one hand to operate the movable clamp and the other to pull the clamped implant from the bone in which it is embedded. Other devices are constructed as chisels that are used to pry the implant from the bone. During implant extraction, such devices extend radially outwardly from the implant. As a consequence, they require considerable radial clearance to enable the chisel to be moved about the circumference of the implant during the prying process, and may interfere with surrounding bone or other bodily tissue as they are moved about the implant.

### BRIEF SUMMARY OF THE DISCLOSURE

In accordance with an embodiment there is provided a medical implant extractor comprising a handle, a shank extending from the handle, and a laterally projecting tip at a distal end of the shank. The laterally projecting tip is adapted to engage an undersurface of the implant to be extracted.

The medical implant extractor includes a substantially S-shaped head extending from the shank, wherein the laterally projecting tip is an upturned tip at a distal end of the substantially S-shaped head, and the substantially S-shaped head creates an offset of the upturned tip from the longitudinal axis of the handle and shaft.

In accordance with another exemplary embodiment there is provided a kit including the above-described medical implant extractor and a striking tool for striking the medical implant extractor.

In accordance with the embodiment there is provided a medical implant extractor having an upturned tip at its distal end. As a result, the tip can be moved around the implant to pry same from the bone while requiring minimal radial clearance to do so and while providing clearance with soft bodily tissue adjacent the bone from which the implant is to be extracted. In addition to the aforesaid medical implant extractor, the subject disclosure provides a striking tool for striking the medical implant extractor and dislodging a firmly embedded implant from bone.

Other features and advantages of the subject disclosure will be apparent from the following more detailed description of the exemplary embodiments. US2013282018 discloses instruments and methods are provided for re-positioning and extracting spinal implants in a space between vertebrae. A tip of an extractor may have a dual tip and offset from a longitudinal axis or an extractor may have a single tip with no offset.
EP2644163A1 discloses a surgical tamp for extracting an implanted humeral stem component for a patient's humerus includes an elongated shaft having a proximal end and an opposite, distal end, a strike plate secured to the proximal end of the elongated shaft, and an engagement tip secured to a distal end of the elongated shaft. The proximal end defines a first longitudinal axis, the distal end defines a second longitudinal axis, and the second axis is offset from and parallel to the first axis.
EP2626043A1 discloses a kit comprising: a plurality of two-part intervertebral disc implants having different sizes; a plurality of implant dispensers, each said dispenser holding together one of said two-part intervertebral disc implants as a single implantable unit, wherein each said implant dispenser has indicia corresponding to the size of said intervertebral disc implant held by said implant dispenser.
US2012004664A1 discloses extraction instruments and systems for use in revision arthroplasty are provided. The extraction instruments are shaped and configured to fit within the typically tight confines in which revision arthroplasty is performed, and to enhance the surgeon's control over the axis, quantum and speed of the force that is required be applied to disrupt a bone-to-prosthesis bond.
US9402740B1 discloses an intervertebral implant for implantation between an upper vertebral body and a lower vertebral body is disclosed. It includes a body portion through which a first and second pathway pass. A first arcuate anchor is movable in an arcuate path through the first pathway and a second arcuate anchor is movable in an arcuate path through the second pathway. A locking member is movable between an open position and a closed position to either block or allow the withdrawal of the first and second arcuate anchors.

### BRIEF DESCRIPTION OF THE SEVERAL VIEWS OF THE DRAWINGS

The foregoing summary, as well as the following detailed description of the exemplary embodiments of the subject disclosure, will be better understood when read in conjunction with the appended drawings. For the purpose of illustrating the present disclosure, there are shown in the drawings exemplary embodiments. It should be understood, however, that the subject application is not limited to the precise arrangements and instrumentalities shown.
FIG. 1 is a perspective view of a medical implant extractor in accordance with an exemplary embodiment of the subject disclosure;
FIG. 2 is a side view of the medical implant extractor of FIG. 1;
FIG. 3 is a front end elevation view of the medical implant extractor of FIG. 1;
FIG. 4 is a side view of a striking tool suitable for striking the medical implant extractor of FIG. 1;
FIG. 5 is an enlarged, partial cross-sectional view of a hammer head of the striking tool of FIG. 4;
FIG. 6 is a front elevational view of the medical implant extractor of FIG. 1 received within the striking tool of FIG. 4;
FIG. 7 is a view of the medical implant extractor of FIG. 1 as it may be positioned when extracting a medical implant from a bone;
FIG. 8 is a side view of another medical implant extractor not forming part of the present invention;
FIG. 9 is a side view of another medical implant extractor not forming part of the present invention;
FIG. 10 is a view of the medical implant extractor of FIG. 8 as it may be positioned when extracting a medical implant from a bone; and
FIG. 11 is a view of the medical implant extractor of FIG. 9 as it may be positioned when extracting a medical implant from a bone.

### DETAILED DESCRIPTION OF THE DISCLOSURE

Reference will now be made in detail to the various exemplary embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject application in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art.

Throughout the subject application, various aspects thereof can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the subject disclosure can be practiced without one or more of the specific features or advantages of a particular exemplary embodiment. In other instances, additional features and advantages may be recognized in certain embodiments that may not be present in all exemplary embodiments of the present disclosure.

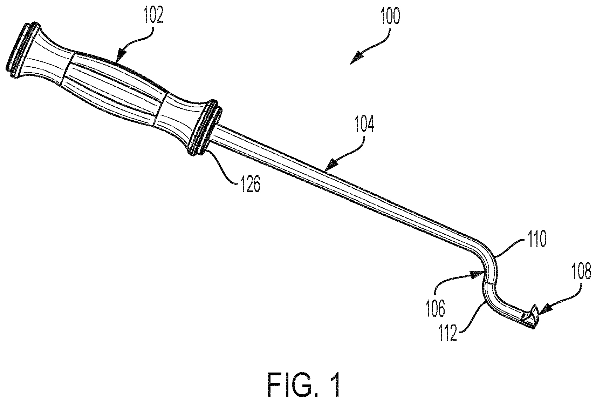

Referring now to the drawings, FIG. 1 illustrates a medical implant extractor 100 constructed in accordance with the subject disclosure. The medical implant extractor includes a handle 102, a shank 104 extending from the handle, and a substantially S-shaped head 106 extending from the shank. The substantially S-shaped head includes an upturned tip 108 at a distal end of the shank.

The handle 102 may be constructed with any ergonomically suitable shape to provide a secure grip by a user, e.g., a surgeon. The handle may further be formed from or surrounded by grip enhancing material to promote a firm grip by a user as well as be sterilizable.

The shank 104, the substantially S-shaped head 106 and upturned tip may be formed from any substantially rigid material including, without limitation, metal such as stainless steel, composite, or reinforced plastic, which is capable of withstanding the tensile forces experienced when extracting an implant, e.g., a glenosphere implant, a glenoid implant, or other implant from bone.

The substantially S-shaped head 106 includes a first curved section 110 having an arc of about 90 degrees and a second curved section 112 extending from the first curved section and having an arc of about 90 degrees. While the first and second curved sections preferably include arcs of about 90 degrees, they could alternatively be more or less, for example, 60, 65, 70, 75, 80, 85, 95, 100, 105, 110, 115, and 120 degrees. Moreover, the substantially S-Shaped head can alternatively be made from non-curved segments such as the plurality of linear segments, e.g., forming a substantially "Z" shaped head.

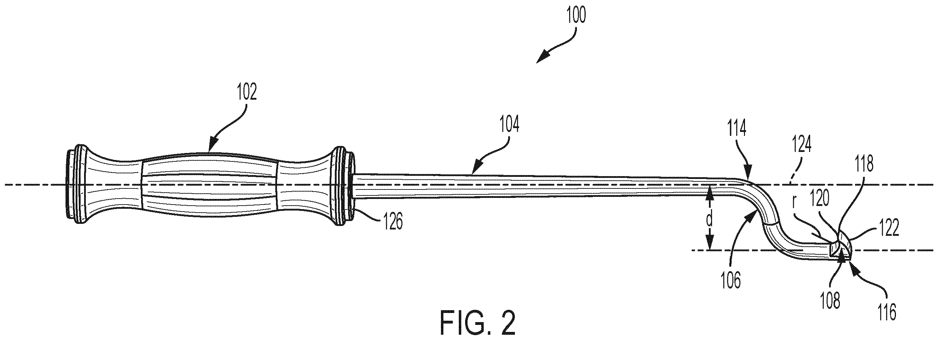

Referring to FIG. 2, a proximal end 114 of the substantially S-shaped head extending from the shank is laterally spaced from a distal end 116 of the substantially S-shaped head a distance "d" of about 12 mm to 52 mm. Further, the proximal end 114 is distally or axially spaced from the distal end 116 of the head.

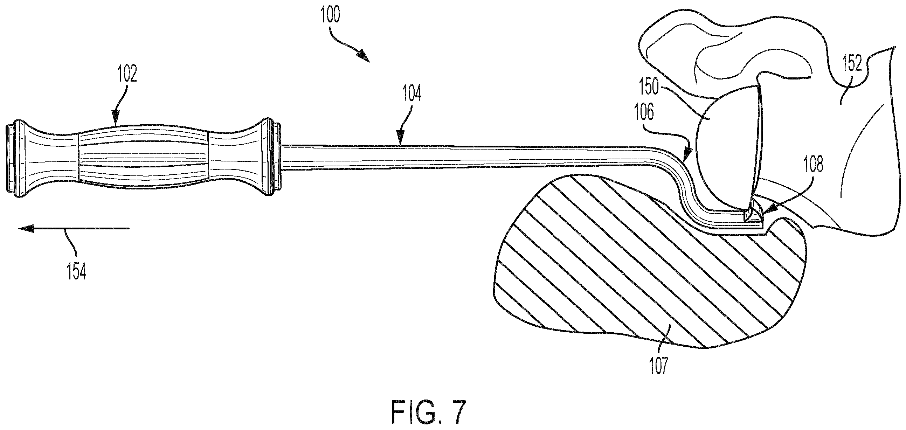

Still referring to FIG. 2, the upturned tip 108 can be substantially ridge-shaped having a peak 118, a proximally facing curved face side 120 and distally facing curved face side 122. As will be described in greater detail in connection with FIG. 7, the upturned tip 108 is configured to be inserted beneath a medical implant to be extracted. The proximally facing curved face side 120 is preferably a concave curved surface. According to an aspect, the proximally facing curved face side 120 of the upturned tip includes a radius of curvature "r" of about 1 mm to 8 mm. The distally facing curved face side 122 may include a similar curvature or it may have a straight profile or chamfer. Importantly, the upturned tip should have a low or narrow profile so as not to interfere with the tissue behind the implant yet be have enough thickness for suitable strength. Overall, the upturned tip 108 is offset from a longitudinal axis 124 of the shank and handle.

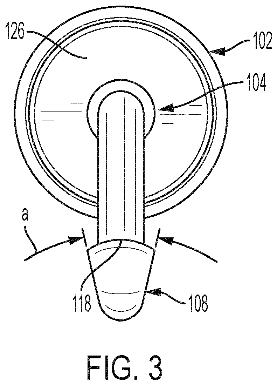

As most clearly shown in FIG. 3, the peak 118 of the upturned tip 108 can be a curved peak having an arc length "a" of about 6 mm to 25 mm. The curved peak may be curved along a plane substantially transverse to the longitudinal axis of the shaft.

Referring to FIGS. 1 through 3, the handle 102 may include a distally facing strike plate 126 that circumscribes the shank 104. The strike plate is preferably formed of hard material e.g. a metal, a composite or a hard plastic. The function of the strike plate 126 is described hereinafter.

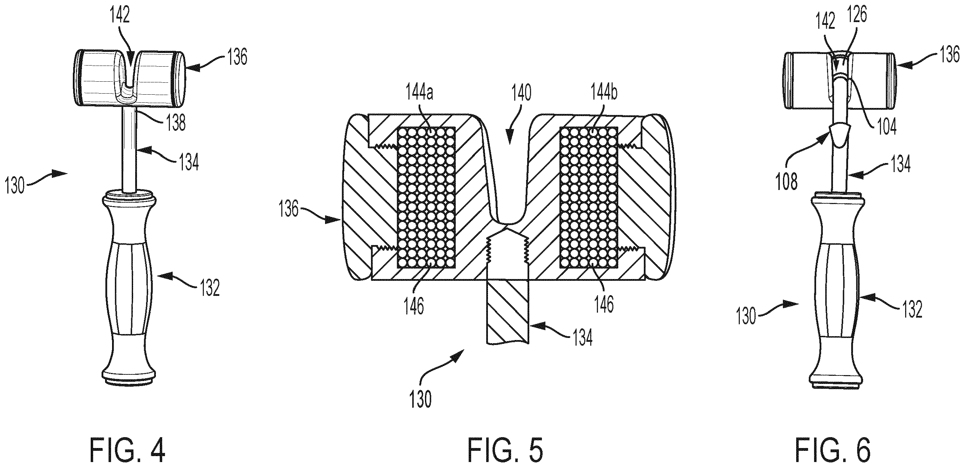

Referring to FIG. 4, there is shown an exemplary striking tool 130 for striking the medical implant extractor 100 and dislodging an embedded implant from bone. The striking tool can comprise a handle 132 and a shaft 134 extending from the handle. The striking tool can further include a hammer head 136 at a distal end 138 of the shaft. The hammer head 136 can comprise a slot 142 about its midportion for receiving the shank 104 of the medical implant extractor 100, as shown in FIG. 6.

As most clearly shown in FIG. 5, the hammer head 136 can include a chamber 144a housing a plurality of weights 146. According to an aspect, the hammer head can include a pair of first and second chambers 144a, 144b each housing a plurality of weights 146. The slot 142 may be positioned between the first and second chambers. According to an aspect, the plurality of weights may comprise metal shot, but can alternatively be any material capable of weighting the hammer head 136 to effectively enhance the force exerted by the hammer head against the medical implant extractor strike plate 126 when the hammer head is impacted against the strike plate, as described below.

Referring to FIG. 7, there is shown the medical implant extractor 100 as it would be positioned when extracting a medical implant 150 from a bone 152. In the illustrated example, medical implant 150 is a glenosphere implant and bone 152 is the scapula. In the alternative, the medical implant extractor 100 may be effectively used to extract a glenoid implant or other medical implant from other bones. As shown in FIG. 7, the user maneuvers the S-shaped head 106 past soft bodily tissue 107 until upturned tip 108 is placed behind a portion of the posterior interface of the medical implant to be extracted. Thereafter, the user pulls on the handle 102 in the direction of arrow 154 thereby exerting extraction force on the medical implant 150 by the upturned tip 108. Assuming the medical implant begins to dislodge under the extraction force exerted by the upturned tip, the user moves the upturned tip behind another portion of the circumference of the medical implant and once again pulls on the handle 102. The user continues this process until the medical implant is dislodged from the bone.

Moreover, the upturned tip 108 can be used for cutting or chipping away bone from an implant as well as cutting or clearing away other bodily tissue in the immediate region of the implant.

If at any time the user experiences resistance to dislodgement of the medical implant that cannot be overcome by simply pulling on the handle 102, the user can deploy the striking tool 130 shown in FIGS. 4 through 6 to strike the medical implant extractor 100. In particular, the user can place the shaft 104 of the medical implant extractor in the slot 142 of the striking tool hammer head 136. Thereafter, while maintaining the upturned tip 108 of the medical implant extractor behind the implant 150, the user holds the handle 132 of the striking tool 130 and forcibly strikes the medical implant extractor strike plate 126 with the hammer head 136. The user can do this as many times as necessary to separate the medical implant 150 from the bone in which it is embedded.

The offset created by the substantially S-shaped head of the medical implant extractor 100 enables the longitudinal axis 124 of the handle and shaft to extend substantially axially with respect to the implant during extraction. As a result, the upturned tip 108 can be moved around the implant to pry same from the bone while requiring minimal radial clearance to do so, thereby avoiding interference with surrounding bone and other bodily tissue. The striking tool 130 may effectively dislodge a firmly embedded implant from bone when struck against the strike plate 126 of the medical implant extractor.

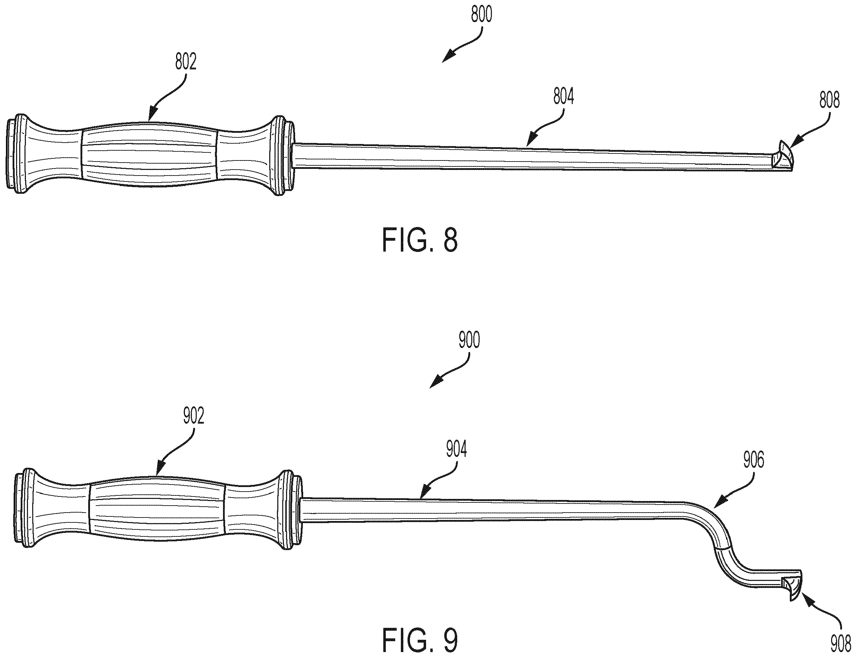

Referring to FIGS. 8 and 9, there are shown medical implant extractors 800 and 900, respectively, both of which do not form part of the present invention. Medical implant extractors 800, 900 are constructed similar to medical implant extractor 100. Accordingly, only those aspects of the medical implant extractors 800, 900 that depart materially in structure and/or function from their counterparts in medical implant extractor 100, or are otherwise necessary for a proper understanding of the subject disclosure, will be discussed in detail.

Referring to FIG. 8, there is shown a medical implant extractor 800. comprising a handle 802 and a shank 804 extending from the handle, wherein the shank is straight throughout its length. A laterally projecting tip 808 extends proud from a distal end of the shank. Tip 808 can be constructed substantially similarly to tip 108 discussed above.

Referring to FIG. 9, there is shown a medical implant extractor 900 comprising a handle 902, a shank 904 extending from the handle, and a substantially S-shaped head 906 extending from the shank. The substantially S-shaped head includes a downturned tip 908 at a distal end of the shank. Tip 908 can be constructed substantially similarly to tip 108 discussed above. Similar to the upturned tip 108 of medical implant extractor 100, the laterally projecting tip 808 of the medical implant extractor 800 and the downturned tip 908 of the medical implant extractor 900 can be used for cutting or chipping away bone from an implant as well as cutting or clearing away other bodily tissue in the immediate region of the implant.

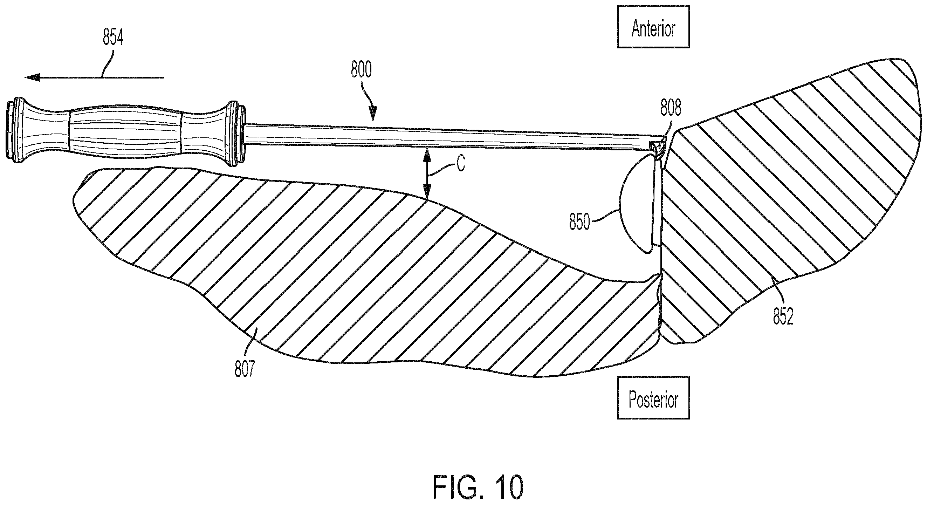

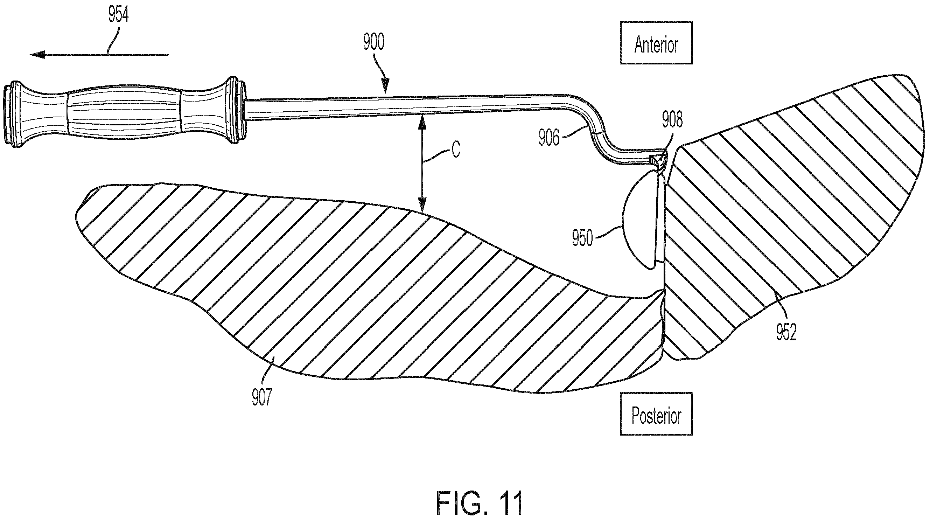

FIGS. 10 and 11 respectively illustrate use of the medical implant extractors 800 and 900 of FIGS. 8 and 9 during a medical implant extraction procedure. The medical implant extractors 800 and 900 may be particularly useful for accessing the anterior side of a medical implant 850 or 950 such as, for example, a glenosphere implant.

Referring initially to FIG. 10, the straight shaft medical implant extractor 800 is shown with the laterally projecting tip 808 of the extractor positioned behind the implant 150 at an anterior interface of the implant 850 with bone 852. With the laterally projecting tip 808 so positioned, a user may exert pulling force in the direction of arrow 854 to dislodge the implant from the bone 852. If necessary, a user may employ a striking tool such as striking tool 130 (FIGS. 4-6) to enhance the dislodging force exerted by the extractor 800.

Significantly, the construction of the straight shaft medical implant extractor 800 provides anterior clearance "C" between soft bodily tissue 807 and the extractor such that the extractor does not interfere with the soft bodily tissue during the extraction process.

Referring to FIG. 11, the medical implant extractor 900 with the substantially S-shaped head 906 and downturned tip 908 is deployed in a manner similar to the medical implant extractor 800. That is, the downturned tip 908 of the extractor is positioned behind the implant 950 at an anterior interface of the implant 150 with bone 952. With the downturned tip 808 so positioned, a user may exert pulling force in the direction of arrow 954 to dislodge the implant from the bone 952. If necessary, a user may employ a striking tool such as striking tool 130 (FIGS. 4-6) to enhance the dislodging force exerted by the extractor 900.

Similar to the medical implant extractor 800, medical implant extractor 900 provides anterior clearance "C" between soft bodily tissue 907 and the extractor such that the extractor does not interfere with the soft bodily tissue. However, because of the substantially S-shaped head 906, the clearance between the extractor 900 and the soft bodily tissue 907 is greater than the corresponding clearance afforded by the straight shaft medical implant extractor 800. Consequently, the likelihood of interference with the soft bodily tissue is even further reduced.

## Claims

1. A medical implant extractor (100) comprising a handle (102), a shank (104) extending from the handle and a substantially S-shaped head (106) extending from the shank,
**characterised by**:
a single upturned projecting tip (108) at a distal end of the substantially S-shaped head (106) extending towards a longitudinal axis (124) of the handle (102),
wherein the single projecting tip (108) is offset from the longitudinal axis (124).

2. The medical implant extractor (100) of claim 1 wherein the single projecting tip (108) is offset from the longitudinal axis (124) about 12 mm to 52 mm.

3. The medical implant extractor (100) of claim 1, wherein the substantially S-shaped head (106) includes a first curved section (110) having an arc of about 90 degrees and a second curved section (112) extending from the first curved section having an arc of about 90 degrees, wherein a proximal end (114) of the substantially S-shaped head (106) extending from the shank (104) is spaced laterally and axially from a distal end (116) of the substantially S-shaped head, or wherein a proximal end (114) of the substantially S-shaped head (106) extending from the shank (104) is laterally spaced from a distal end (116) of the substantially S-shaped head about 12 mm to 52 mm.

4. The medical implant extractor (100) of claim 1, wherein the single projecting tip (108) is substantially ridge-shaped having a peak (118), a proximally facing curved face side (120) and a distally facing curved face side (122).

5. The medical implant extractor (100) of claim 4, wherein the proximally facing curved face side (120) includes a radius of curvature of about 1 mm to 8 mm, or wherein the peak (118) is a curved peak, or wherein the peak is a curved peak having an arc length of about 6 mm to 25 mm.

6. The medical implant extractor (100) of claim 1, wherein the handle (102) includes a distally facing strike plate (126).

7. The medical implant extractor (100) of claim 6, wherein the distally facing strike plate (126) circumscribes the shank (104).

8. A medical implant extractor kit comprising:
the medical implant extractor (100) of claims 1-7; and
a striking tool (130) for striking the medical implant extractor (100).

9. The medical implant extractor kit of claim 8, wherein the striking tool (130) comprises:
a handle (132);
a shaft (134) extending from the handle (132); and
a hammer head (136) at a distal end (138) of the shaft (134), the hammer head (136) comprising a slot (142) about its midportion for receiving the shank (104) of the medical implant extractor (100).

10. The medical implant extractor kit of claim 9, wherein the hammer head (136) includes a chamber (144a) housing a plurality of weights (146), or wherein the hammer head (136) includes a pair of chambers (144a, 144b) each housing a plurality of weights (146).

11. The medical implant extractor kit of claim 10, wherein the hammer head (136) includes a first and second chambers (144a, 144b) each housing a plurality of weights (146), and wherein the slot (142) is positioned between the first and second chambers (144a, 144b).

12. The medical implant extractor kit of claim 11, wherein the weights (146) comprise metal shot.

## Patentansprüche

1. Extraktor (100) eines medizinischen Implantats, umfassend einen Griff (102), einen Schaft (104), der sich von dem Griff erstreckt, und einen im Wesentlichen S-förmigen Kopf (106), der sich von dem Schaft erstreckt, **gekennzeichnet durch:**
eine einzelne aufgeklappte vorstehende Spitze (108) an einem distalen Ende des im Wesentlichen S-förmigen Kopfes (106), die sich zu einer Längsachse (124) des Griffs (102) hin erstreckt,
wobei die einzelne vorstehende Spitze (108) von der Längsachse (124) versetzt ist.

2. Extraktor (100) des medizinischen Implantats nach Anspruch 1, wobei die einzelne vorstehende Spitze (108) von der Längsachse (124) um 12 mm bis 52 mm versetzt ist.

3. Extraktor (100) des medizinischen Implantats nach Anspruch 1, wobei der im Wesentlichen S-förmige Kopf (106) einen ersten gekrümmten Bereich (110), der einen Bogen von etwa 90 Grad aufweist, und einen zweiten gekrümmten Bereich (112) einschließt, der sich von dem ersten gekrümmten Bereich erstreckt, der einen Bogen von etwa 90 Grad aufweist, wobei ein proximales Ende (114) des im Wesentlichen S-förmigen Kopfes (106), das sich von dem Schaft (104) erstreckt, von einem distalen Ende (116) des im Wesentlichen S-förmigen Kopfes seitlich und axial beabstandet ist, oder wobei ein proximales Ende (114) des im Wesentlichen S-förmigen Kopfes (106), das sich von dem Schaft (104) erstreckt, von einem distalen Ende (116) des im Wesentlichen S-förmigen Kopfes um 12 mm bis 52 mm seitlich beabstandet ist.

4. Extraktor (100) des medizinischen Implantats nach Anspruch 1, wobei die einzelne vorstehende Spitze (108) im Wesentlichen rippenförmig ist, die einen Peak (118), eine proximal zugewandte gekrümmte Stirnseite (120) und eine distal zugewandte gekrümmte Stirnseite (122) aufweist.

5. Extraktor (100) des medizinischen Implantats nach Anspruch 4, wobei die proximal zugewandte gekrümmte Stirnseite (120) einen Krümmungsradius von etwa 1 mm bis 8 mm einschließt, oder wobei der Peak (118) ein gekrümmter Peak ist, oder wobei der Peak ein gekrümmter Peak ist, der eine Bogenlänge von etwa 6 mm bis 25 mm aufweist.

6. Extraktor (100) des medizinischen Implantats nach Anspruch 1, wobei der Griff (102) eine distal zugewandte Schließplatte (126) einschließt.

7. Extraktor (100) des medizinischen Implantats nach Anspruch 6, wobei die distal zugewandte Schließplatte (126) den Schaft (104) begrenzt.

8. Extraktorkit des medizinischen Implantats, umfassend:
den Extraktor (100) des medizinischen Implantats nach Anspruch 1 bis 7; und
ein Schlagwerkzeug (130) zum Schlagen auf den Extraktor (100) des medizinischen Implantats.

9. Extraktorkit des medizinischen Implantats nach Anspruch 8, wobei das Schlagwerkzeug (130) umfasst:
einen Griff (132);
einen Stiel (134), die sich von dem Griff (132) erstreckt, und
einen Hammerkopf (136) an einem distalen Ende (138) des Stiels (134), der Hammerkopf (136) umfassend einen Schlitz (142) um seinen Mittelabschnitt herum zum Aufnehmen des Schafts (104) des Extraktors (100) des medizinischen Implantats.

10. Extraktorkit des medizinischen Implantats nach Anspruch 9, wobei der Hammerkopf (136) eine Kammer (144a) einschließt, die eine Vielzahl von Gewichtungen (146) aufnimmt, oder wobei der Hammerkopf (136) ein Paar Kammern (144a, 144b) einschließt, die jeweils eine Vielzahl von Gewichtungen (146) aufnehmen.

11. Extraktorkit des medizinischen Implantats nach Anspruch 10, wobei der Hammerkopf (136) eine erste und eine zweite Kammer (144a, 144b) einschließt, die jeweils eine Vielzahl von Gewichtungen (146) aufnehmen, und wobei der Schlitz (142) zwischen der ersten und der zweiten Kammer (144a, 144b) positioniert ist.

12. Extraktorkit des medizinischen Implantats nach Anspruch 11, wobei die Gewichtungen (146) Schrot umfassen.

## Revendications

1. Extracteur d'implants médicaux (100) comprenant une poignée (102), une tige (104) s'étendant à partir de la poignée et une tête sensiblement en forme de S (106) s'étendant à partir de la tige, **caractérisé par** :
une pointe unique faisant saillie tournée vers le haut (108) au niveau d'une extrémité distale de la tête sensiblement en forme de S (106) s'étendant vers un axe longitudinal (124) de la poignée (102),
dans lequel la pointe unique faisant saillie (108) est décalée de l'axe longitudinal (124).

2. Extracteur d'implants médicaux (100) selon la revendication 1, dans lequel la pointe unique faisant saillie (108) est décalée de l'axe longitudinal (124) d'environ 12 mm à 52 mm.

3. Extracteur d'implants médicaux (100) selon la revendication 1, dans lequel la tête sensiblement en forme de S (106) comporte une première section incurvée (110) ayant un arc d'environ 90 degrés et une seconde section incurvée (112) s'étendant à partir de la première section incurvée ayant un arc d'environ 90 degrés, dans lequel une extrémité proximale (114) de la tête sensiblement en forme de S (106) s'étendant à partir de la tige (104) est espacée latéralement et axialement à partir d'une extrémité distale (116) de la tête sensiblement en forme de S, ou dans lequel une extrémité proximale (114) de la tête sensiblement en forme de S (106) s'étendant à partir de la tige (104) est latéralement espacée d'une extrémité distale (116) de la tête sensiblement en forme de S d'environ 12 mm à 52 mm.

4. Extracteur d'implants médicaux (100) selon la revendication 1, dans lequel la pointe unique faisant saillie (108) est sensiblement en forme de crête ayant un pic (118), un côté de face incurvée orienté proximalement (120) et un côté de face incurvée orienté distalement (122).

5. Extracteur d'implants médicaux (100) selon la revendication 4, dans lequel le côté de face incurvée orienté proximalement (120) comporte un rayon de courbure d'environ 1 mm à 8 mm, ou dans lequel le pic (118) est un pic incurvé, ou dans lequel le pic est un pic incurvé ayant une longueur d'arc d'environ 6 mm à 25 mm.

6. Extracteur d'implants médicaux (100) selon la revendication 1, dans lequel la poignée (102) comporte une plaque de frappe orientée distalement (126).

7. Extracteur d'implants médicaux (100) selon la revendication 6, dans lequel la plaque de frappe orientée distalement (126) entoure la tige (104).

8. Kit d'extraction d'implants médicaux comprenant :
l'extracteur d'implants médicaux (100) selon les revendications 1 à 7 ; et
un outil de frappe (130) permettant de frapper l'extracteur d'implants médicaux (100).

9. Kit d'extraction d'implants médicaux selon la revendication 8, dans lequel l'outil de frappe (130) comprend :
une poignée (132) ;
un arbre (134) s'étendant à partir de la poignée (132) ; et
une tête de marteau (136) au niveau d'une extrémité distale (138) de l'arbre (134), la tête de marteau (136) comprenant une fente (142) autour de sa partie médiane destinée à recevoir la tige (104) de l'extracteur d'implants médicaux (100).

10. Kit d'extraction d'implants médicaux selon la revendication 9, dans lequel la tête de marteau (136) comporte une chambre (144a) logeant une pluralité de poids (146), ou dans lequel la tête de marteau (136) comporte une paire de chambres (144a, 144b) logeant chacune une pluralité de poids (146).

11. Kit d'extraction d'implants médicaux selon la revendication 10, dans lequel la tête de marteau (136) comporte une première et une seconde chambre (144a, 144b) logeant chacune une pluralité de poids (146), et dans lequel la fente (142) est positionnée entre les première et seconde chambres (144a, 144b).

12. Kit d'extraction d'implants médicaux selon la revendication 11, dans lequel les poids (146) comprennent une grenaille métallique.
